(19) Europäisches Patentamt
European Patent Office
Office européen des brevets

(11) Veröffentlichungsnummer : **0 023 713**
**B1**

(12) # EUROPÄISCHE PATENTSCHRIFT

(45) Veröffentlichungstag der Patentschrift : **09.03.83**

(51) Int. Cl.$^3$ : **C 07 J 5/00**, A 61 K 31/57

(21) Anmeldenummer : **80104561.8**

(22) Anmeldetag : **01.08.80**

(54) **Neue Derivate des 9-Chlorprednisolons, ihre Herstellung und Verwendung.**

(30) Priorität : **06.08.79 DE 2932165**

(43) Veröffentlichungstag der Anmeldung : **11.02.81 Patentblatt 81/06**

(45) Bekanntmachung des Hinweises auf die Patenterteilung : **09.03.83 Patentblatt 83/10**

(84) Benannte Vertragsstaaten : **AT BE CH DE FR GB IT LI LU NL SE**

(56) Entgegenhaltungen : **DE A 2 645 105**

**Die Akte enthält technische Angaben, die nach dem Eingang der Anmeldung eingereicht wurden und die nicht in dieser Patentschrift enthalten sind.**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Annen, Klaus, Dr.**
**Seegefelder Strasse 194**
**D-1000 Berlin 20 (DE)**
Erfinder : **Laurent, Henry, Dr.**
**Glambecker Weg 21**
**D-1000 Berlin 28 (DE)**
Erfinder : **Hofmeister, Helmut, Dr.**
**Weislingenstrasse 4**
**D-1000 Berlin 28 (DE)**
Erfinder : **Wiechert, Rudolf, Prof.**
**Petzower Strasse 8a**
**D-1000 Berlin 39 (DE)**
Erfinder : **Kapp, Joachim-Friedrich, Dr.**
**Ludolfinger Weg 51**
**D-1000 Berlin 28 (DE)**
Erfinder : **Wendt, Hans, Dr.**
**Ernst-Ring-Strasse 14**
**D-1000 Berlin 38 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 023 713 B1

# Neue Derivate des 9-Chlorprednisolons, ihre Herstellung und Verwendung.

Die Erfindung betrifft den in den Patentansprüchen gekennzeichneten Gegenstand.

Gegenüber den aus der deutschen Offenlegungsschrift 26 45 105 vorbekannten 9α-Chlorprednisolon-Derivaten zeichnen sich die erfindungsgemässen Verbindungen der allgemeinen Formel I gemäss Anspruch 1 durch eine verstärkte Dissoziation von erwünschter topischer antiinflammatorischer Wirksamkeit und unerwünschten Kortikoidnebenwirkungen aus, wie man beispielsweise mit Hilfe des bekannten Vasokonstriktionstests einerseits und des bekannten Thymolysetests, Nebennierensuppressionstests oder Hautreissversuchs andererseits nachweisen kann.

Das erfindungsgemässe Verfahren zur Herstellung der 9α-Chlorprednisolon-Derivate wird unter den aus der deutschen Offenlegungsschrift 26 45 105 vorbekannten Verfahrensbedingungen durchgeführt.

Die neuen 9-Chlorprednisolon-Derivate eignen sich in Kombination mit den in der galenischen Pharmazie üblichen Trägermitteln zur lokalen Behandlung von Kontaktdermatitis, Ekzemen der verschiedensten Art, Neurodermatosen, Erythrodermie, Verbrennungen, Pruritis vulvae et ani, Rosacea, Erythematodes cutaneus, Psoriasis, Lichen ruber planus et verrucosus und ähnlichen Hauterkrankungen.

Die Herstellung der Arzneimittelspezialitäten erfolgt in üblicher Weise, indem man die Wirkstoffe mit geeigneten Zusätzen in die gewünschte Applikationsform, wie zum Beispiel: Lösungen, Lotionen, Salben, Cremes oder Pflaster, überführt. In den so formulierten Arzneimitteln ist die Wirkstoffkonzentration von der Applikationsform abhängig. Bei Lotionen und Salben wird vorzugsweise eine Wirkstoffkonzentration von 0,001 % bis 1 % verwendet.

Darüberhinaus sind die neuen 9-Chlorprednisolon-Derivate gegebenenfalls in Kombination mit den üblichen Trägermitteln und Hilfsstoffe auch gut zur Herstellung von Inhalationsmitteln geeignet, welche zur Therapie allergischer Erkrankungen der Atemwege, wie zum Beispiel des Bronchialasthmas oder der Rhinitis verwendet werden können.

Die nachfolgenden Beispiele dienen zur Erläuterung des erfindungsgemässen Verfahrens.

## Beispiel 1

a) Eine Suspension von 6 g 21-Acetoxy-17α-hydroxy-1.4.9(11)-pregnatrien-3.20-dion in 100 ml Diethylenglykoldimethylether werden mit 20 g N,N-Dimethylaminopyridin und 30 ml Trimethylessigsäureanhydrid versetzt und 90 Stunden bei einer Badtemperatur von 80 °C gerührt. Das Reaktionsgemisch wird mit Methylenchlorid verdünnt und mit 2N Salzsäure gewaschen. Nach der Wasserdampfdestillation extrahiert man mit Methylenchlorid, trocknet über Natriumsulfat und isoliert nach Eindampfen 7.8 g 21-Acetoxy-17α-trimethylacetoxy-1.4.9(11)-pregnatrien-3.20-dion.

b) 7.3 g des so erhaltenen Rohprodukts werden in 100 ml Dioxan suspendiert und mit 6.6 g N.-Chlorsuccinimid versetzt. Dann tropft man innerhalb von 10 Minuten bei 20 °C 50 ml einer 10 %igen wässrigen Perchlorsäurelösung in die Mischung, rührt sie 3 Stunden lang bei 20 °C und giesst sie in eine Lösung von 3.0 g Natriumhydrogensulfit in 500 ml Wasser. Das ausgeschiedene Produkt wird abgesaugt und an 700 g Kieselgel mit einem Methylenchlorid-Aceton-Gradienten (0-10 % Aceton) gereinigt. Ausbeute 3.4 g 21-Acetoxy-9α-chlor-11β-hydroxy-17α-trimethylacetoxy-1.4.-pregnadien-3.20-dion. Schmelzpunkt 249 °C (Zers.).

## Beispiel 2

a) 5.0 g 17α-hydroxy-21-propionyloxy-1.4.9(11)-pregnatrien-3.20-dion in 80 ml Diethylenglykoldimethylether werden analog Beispiel 1a) mit 18 g N.N-Dimethylaminopyridin und 25 ml Trimethylessigsäureanhydrid umgesetzt und aufgearbeitet. Man isoliert 6.7 g 21-Propionyloxy-17α-trimethylacetoxy-1.4.9(11)-pregnatrien-3.20-dion.

b) Analog Beispiel 1b) werden 6.5 g des obigen Rohproduktes mit N-Chlorsuccinimid behandelt. Das Reaktionsprodukt wird an 700 g Kieselgel mit einem Methylenchlorid-Aceton-Gradienten (0-10 % Aceton) gereinigt. Ausbeute 3.1 g 9α-Chlor-11β-hydroxy-21-propionyloxy-17α-trimethylacetoxy-1.4-pregnadien-3.20-dion. Schmelzpunkt 247 °C.

## Beispiel 3

a) Eine Lösung von 6.0 g 21-Butyryloxy-17α-hydroxy-1.4.9(11) pregnatrien-3.20-dion in 100 ml Diethylenglykoldimethylether werden analog Beispiel 1a) mit 20 g N.N-Dimethylaminopyridin und 30 ml Trimethylessigsäureanhydrid behandelt. Nach der Aufarbeitung erhält man 7.4 g 21-Butyryloxy-17α-trimethylacetoxy-1.4.9(11)-pregnatrien-3.20-dion.

b) Analog Beispiel 1b) werden 7.1 g obigen Rohprodukts mit N-Chlorsuccinimid umgesetzt. Das Reaktionsprodukt wird an 700 g Kieselgel mit einem Methylenchlorid-Aceton-Gradienten (0-10 % Aceton) gereinigt. Ausbeute 2.9 g 21-Butyryloxy-9α-chlor-11β-hydroxy-17α-trimethylacetoxy-1.4.pregnadien-3.20-dion. Schmelzpunkt 261 °C.

Beispiel 4

a) Unter den Bedingungen des Beispiels 1a) werden 5.0 g 17α-Hydroxy-21-valeryloxy-1.4.9(11)-pregnatrien-3.20-dion in 80 ml Diethylenglykoldimethylether mit 18 g N.N-Dimethylaminopyridin und 25 ml Trimethylessigsäureanhydrid umgesetzt und aufgearbeitet. Man erhält 6.4 g 17α-Trimethylacetoxy-21-valeryloxy-1.4.9(11)-pregnatrien-3.20-dion.

b) 6.0 g des obigen Rohproduktes werden analog Beispiel 1b) mit N-Chlorsuccinimid behandelt. Man reinigt das Reaktionsprodukt an 700 g Kieselgel mit einem Methylenchlorid-Aceton-Gradienten (0.10 % Aceton). Ausbeute 3.4 g 9α-Chlor-11β-hydroxy-17α-trimethylacetoxy-21-valeryloxy-1.4-pregnadien-3.20-dion.

**Ansprüche**

1. 9-Chlorprednisolon-Derivate der allgemeinen Formel I

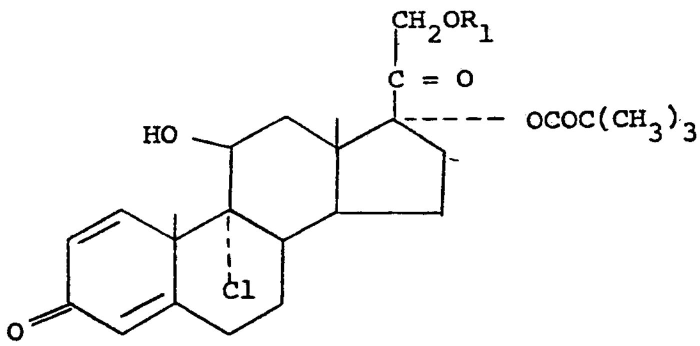

(I)

worin

$R_1$ eine 2 bis 5 Kohlenstoffatome enthaltende 1 Oxoalkylgruppe bedeutet.

2. 21-Acetoxy-9α-chlor-11β-hydroxy-17α-trimethylacetoxy-1.4-pregnadien-3.20-dion,

3. 9α-Chlor-11β-hydroxy-21-propionyloxy-17α-trimethylacetoxy-1.4-pregnadien-3.20-dion,

4. 21-Butyryloxy-9α-chlor-11β-hydroxy-17α-trimethylacetoxy-1.4-pregnadien-3.20-dion,

5. 9α-Chlor-11β-hydroxy-17α-trimethyloxy-21-valeryloxy-1.4-pregnadien-3.20-dion,

6. Pharmazeutische Präparate, enthaltend ein 9α-Chlorprednisolon-Derivat gemäss Anspruch 1 bis 5 als Wirkstoff.

7. Verfahren zur Herstellung von 9-Chlorprednisolon-Derivaten der allgemeinen Formel I gemäss Anspruch 1, dadurch gekennzeichnet, dass man in an sich bekannter Weise an eine Verbindung der allgemeinen Formel II,

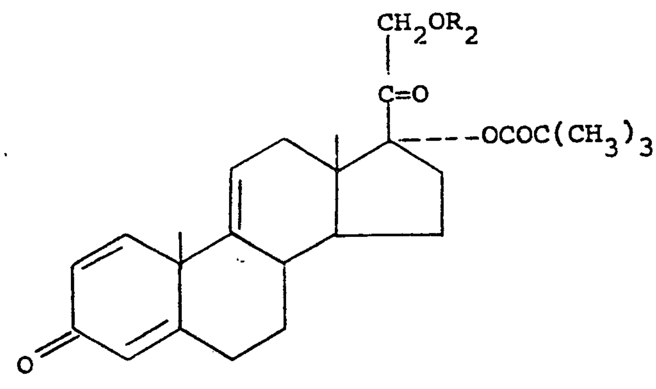

(II)

worin

$R_2$ eine 2 bis 5 Kohlenstoffatome enthaltende 1-Oxoalkylgruppe bedeutet, HO—Cl anlagert.

**Claims**

1. 9-chloroprednisolone derivatives of the general formula I

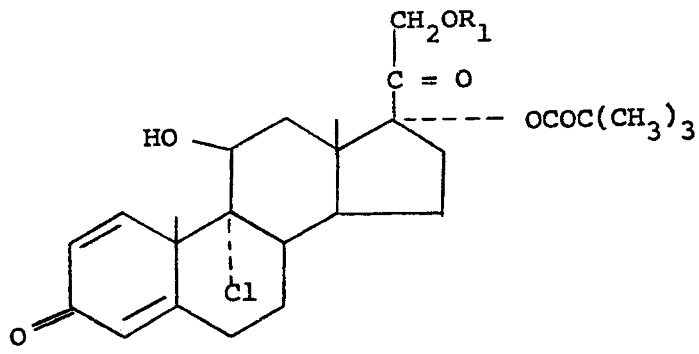

(I)

in which

$R_1$ represents a 1-oxoalkyl group containing from 2 to 5 carbon atoms.

2. 21-acetoxy-9α-chloro-11β-hydroxy-17α-trimethyl-acetoxy-1,4-pregnadiene-3,20-dione.

3. 9α-chloro-11β-hydroxy-21-propionyloxy-17α-trimethyl-acetoxy-1,4-pregnadiene-3,20-dione.

4. 21-butyryloxy-9α-chloro-11β-hydroxy-17α-trimethyl-acetoxy-1,4-pregnadiene-3,20-dione.

5. 9α-chloro-11β-hydroxy-17α-trimethyloxy-21-valeryloxy-1,4-pregnadiene-3,20-dione.

6. Pharmaceutical preparations containing a 9α-chloroprednisolone derivative according to claims 1 to 5 as the active ingredient.

7. Process for the manufacture of 9-chloroprednisolone derivatives of the general formula I according to claim 1, characterised in that HO—Cl is added in a manner known *per se* to a compound of the general formula II

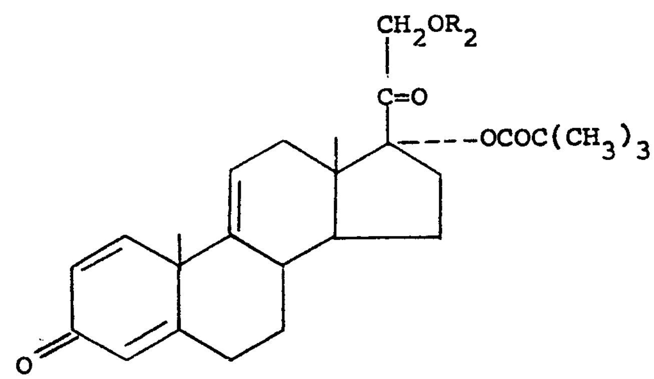

(II)

in which

$R_2$ represents a 1-oxoalkyl group containing from 2 to 5 carbon atoms.

**Revendications**

1. Dérivés de la chloro-9 prednisolone répondant à la formule I

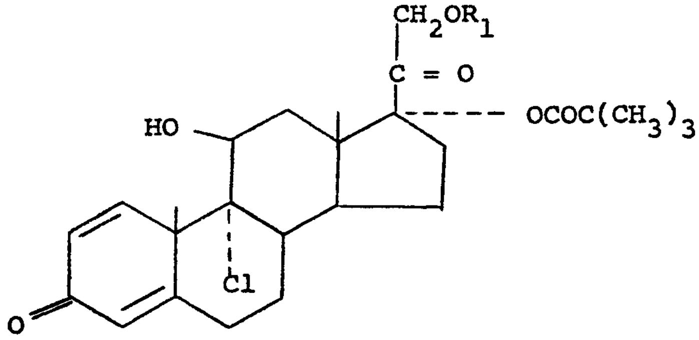

(I)

dans laquelle

$R_1$ représente un radical oxo-1 alkyle contenant de 2 à 5 atomes de carbone.

2. Chloro-9α hydroxy-11β triméthylacétoxy-17α acétoxy-21 prégnadiène-1,4 dione-3,20.

3. Chloro-9α hydroxy-11β triméthylacétoxy-17α propionyloxy-21 prégnadiène-1,4 dione-3,20.

4. Chloro-9α hydroxy-11β triméthylacétoxy-17α butyryloxy-21 prégnadiène-1,4 dione-3,20.

5. Chloro-9α hydroxy-11β triméthylacétoxy-17α valéryloxy-21 prégnadiène-1,4 dione-3,20.

6. Médicament contenant, comme substance active, un dérivé de la chloro-9α prednisolone selon l'une quelconque des revendications 1 à 5.

7. Préparation de dérivés de la chloro-9 prednisolone de formule générale I selon la revendication 1, procédé caractérisé en ce qu'on fixe HO—Cl, de manière connue, sur un composé répondant à la formule générale II

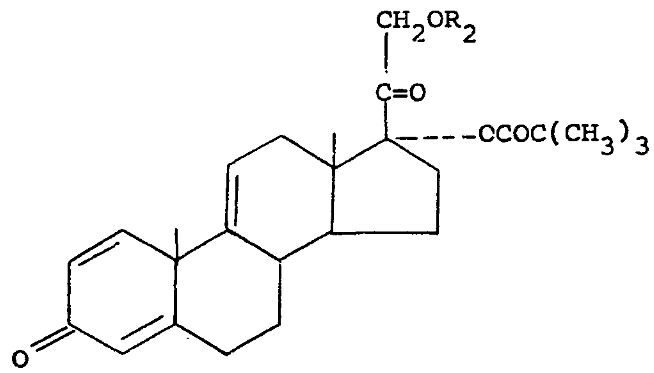

(II)

dans laquelle

$R_2$ représente un radical oxo-1 alkyle contenant de 2 à 5 atomes de carbone.